# EUROPÄISCHE PATENTSCHRIFT

(11) **EP 1 316 554 B1**
(45) Veröffentlichungstag und Bekanntmachung des Hinweises auf die Patenterteilung: **26.04.2006**
(21) Anmeldenummer: 02025980.0
(22) Anmeldetag: 21.11.2002
(51) Int. Cl.: C07D 319/06

(54) **Acetal-Derivate aus 2-Methyl-2secbutyl-1,3-propandiol und ihre Verwendung als Riechstoff**
Acetal derivatives of 2-methyl-2secbutyl-1,3-propandiol and their use as fragrance
Acétals dérivés de 2-méthyl-2secbutyl-1,3-propanediol et leur utilisation comme principe odorant

(30) Priorität: 30.11.2001 DE 10158908
(43) Veröffentlichungstag der Anmeldung: 04.06.2003
(73) Patentinhaber: Celanese Chemicals Europe GmbH, 60439 Frankfurt am Main (DE)
(72) Erfinder: Lappe, Peter, Dr., 46539 Dinslaken (DE); Schmid, Klaus, Dr., 46539 Dinslaken (DE); Söllner, Rolf, Dr., 46535 Dinslaken (DE); Springer, Helmut, 46539 Dinslaken (DE)

(56) Entgegenhaltungen:
- EP-A- 0 276 998
- EP-A- 1 316 553
- JP-A- 54 144 352
- US-A- 3 748 344

## Beschreibung

Die Erfindung betrifft neue Riechstoffe auf Basis cyclischer Acetale, die sich von 2-Methyl-2-sec.butyl-1,3-propandiol als Alkohol- und von 8-Formyltricyclo[5.2.1.0^{2.6}]-decan oder von einem Gemisch aus 8- und 9-Formyltricyclo[5.2.1.0^{2.6}]-decen-3 als Aldehydkomponente ableiten.

In der Parfümerie wurden anfangs ausschließlich natürliche Stoffe als Geruchsträger verwendet. Diese Stoffe stehen jedoch nicht immer in ausreichender Menge und in gleichbleibender Qualität zur Verfügung. Häufig ist ihre Verwendung auch aus ökologischen Gründen nur eingeschränkt möglich und überdies sind sie oft sehr teuer, so dass sich ihr Einsatz als Geruchsgeber für Massenprodukte verbietet. Daher werden heutzutage in großem Umfang synthetische Substanzen als Riechstoffe eingesetzt.

In den letzten Jahren haben neben einer großen Anzahl anderer Verbindungsklassen cyclische Acetale und Ketale als industriell erzeugte Riechstoffe an Bedeutung gewonnen. In einer umfangreichen Arbeit in Angew. Chemie 2000, 112, 3107-3138 wird der Einsatz von cyclischen Acetalen und Ketalen als Ambra-Riechstoffe beschrieben. Beispiele für derartige Verbindungen sind u.a. Acetale und Ketale, die sich von 1-Formyl-2,4-dimethylcyclohexen-3 und 2-Methyl-2-sec.butyl-propan-1,3-diol und von 2-Formylcaran und Aceton ableiten. Ferner werden Dioxolane und 1,3-Dioxane als potentielle Riechstoffe erwähnt. Diese Verbindungen sind aus α-Cedren bzw. aus Campher leicht zugänglich.

In der EP 0 276 998 A2 werden Parfümkompositionen beschrieben, die als Riechstoff mindestens eine Verbindung der allgemeinen Formel 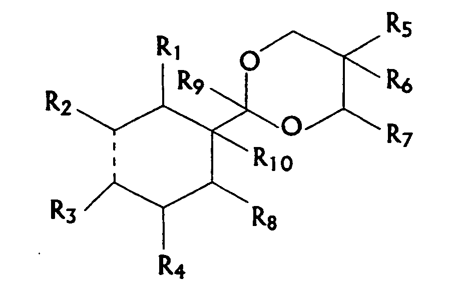 enthalten. In dieser Formel steht die gestrichelte Linie für eine Doppel- oder Einfachbindung innerhalb des Cyclohexanringes. R₁, R₂, R₃ und R₄ bedeuten Wasserstoff oder einen Methylrest. R₅ und R₆ sind Wasserstoff oder ein Alkyl-, ein Cycloalkyl- oder ein Alkenylrest mit 1 bis 4 Kohlenstoffatomen oder ein, gegebenenfalls substituierter, Ring, der bis zu 5 Kohlenstoffatome enthält. R₅ und R₆ können zusammen mit dem Kohlenstoffatom des Dioxanringes einen, gegebenenfalls substituierten, Ring mit bis zu 6 Kohlenstoffatomen bilden. R₇ bedeutet Wasserstoff, Methyl, Ethyl, n-Propyl, Isopropyl, n-Butyl, sec.Butyl, Isobutyl oder t-Butyl. R₈ und R₉ stehen jeweils für Wasserstoff oder den Methylrest. R₁₀ ist Wasserstoff, Methyl oder Isopropyl. Die Gesamtzahl der Kohlenstoffatome liegt im Bereich von 13 bis 20.

Die durch die vorstehende, allgemeine Formel beschriebenen Verbindungen decken einen weiten Riechstoffbereich ab, einige von ihnen besitzen eine überraschende Ambranote.

Die Herstellung der genannten Verbindungen erfolgt in einer ersten Stufe durch Diels-Alder-Reaktion von Dienen mit ungesättigten Aldehyden oder Ketonen, die zu cyclischen Carbonylverbindungen führt. Beispiele für geeignete Diene sind Butadien-1,3 und 2-Methyl-1,3-pentadien. Als Aldehyde oder Ketone kommen z.B. Acrolein, Methacrolein, Crotonaldehyd oder Methylvinylketon in Betracht. In einer zweiten Stufe werden die Carbonylver bindungen mit Diolen wie 2-Methyl-2-propyl-1,3-propandiol, 2-Methyl-2-sec.butyl-1,3-propandiol oder 2-Ethylhexan-1,3-diol zum erwünschten Acetal oder Ketal umgesetzt.

Aus US 3,748,344 sind cyclische Acetale bekannt, in denen die cyclischen Ausgangsaldehyde auf dem gesättigten Norbornan- und auf dem ungesättigten Norbornenrest basieren. Als Diole werden unter anderem 1,3-Diole, darunter auch 2,2-Dimethyl-1,3-propandiol, verwendet. Die bekannten Verbindungen eignen sich zur Herstellung von Riechstoffen.

JP 54 144 352 behandelt die Herstellung von Acetalen durch Umsetzung von 8-Exo-formyl-endotricyclo[5.2.1.0^{2.6}]-decan oder eines Gemisches von 8-Exo-formyl-endotricyclo[5.2.1.0^{2.6}]-deca-3-en und 9-Exo-formyl-endotricyclo[5.2.1.0^{2.6}]-deca-3-en mit Diolen, wie beispielsweise Ethylenglykol, 1,2-Propandiol, 1,3-Propandiol, 2,3-Butandiol, 1,3-Butandiol sowie 1,4-Butandiol. Die bekannten Verbindungen lassen sich zur Herstellung von Duftzusammensetzungen verwenden.

EP-A1-1 316 553 offenbart Riechstoffe auf Basis cyclischer Acetale, die sich von Diolen, unter anderem von 2-Methyl-2-sec.butyl-1,3-propandiol, als Alkoholkomponente und von substituierten Propionaldehyden als Aldehydkomponente ableiten.

Obgleich es bereits eine Vielzahl synthetisch erzeugter Riechstoffe gibt, besteht in der industriellen Praxis weiterhin ein hoher Bedarf an Duftträgern. Das Interesse ist dabei sowohl auf die Entwicklung neuer Duftnoten gerichtet als auch auf die möglichst kostengünstige Herstellung bekannter Riechstoffe, sei es durch verfahrenstechnische Vereinfachung des Syntheseweges oder durch Auswahl preiswerter Rohstoffe. Der vorliegenden Erfindung liegt die Aufgabe zugrunde, beiden Erfordernissen der industriellen Fertigung zu genügen durch Bereitstellung neuer Verbindungen mit bekannten oder neuen Duftnoten, die aus leicht zugänglichen, in ausreichenden Mengen preisgünstig zur Verfügung stehenden Ausgangsstoffen gewonnen werden können. Zusätzlich wird Wert darauf gelegt, dass der Weg zu ihrer Herstellung technisch einfach ist und keine aufwändigen oder spezialisierten Apparaturen erfordert.

Die vorliegende Erfindung betrifft Riechstoffe auf Basis der chemischen Verbindung 2-(Tricyclo[5.2.1.0^{2.6}]-dec-8-yl)-5-methyl-5-(1-methylpropyl)-1,3-dioxan oder auf Basis eines Gemisches bestehend aus 2-(Tricyclo[5.2.1.0^{2.6}]-dec-3-en-8-yl)-5-methyl-5-(1-methylpropyl)-1,3-dioxan und 2-(Tricyclo-[5.2.1.0^{2.6}]-dec-3-en-9-yl)5-methyl-5-(1-methylpropyl)-1,3-dioxan.

Von cyclischen aliphatischen Verbindungen, nämlich von Verbindungen des Cyclohexans und des Cyclohexens, in denen jeweils ein Ringwasserstoffatom durch eine Formylgruppe ersetzt ist, abgeleitete Acetale sind als Riechstoffe bekannt. Dennoch war es nicht zu erwarten, dass auch Acetale auf Basis mehrcyclischer aliphatischer Formylverbindungen, die mehr als sechs Kohlenstoffatome im Ringsystem enthalten, Riechstoffcharakter besitzen. Hierbei ist zu berücksichtigen, dass, ähnlich wie die pharmakologische Wirksamkeit bestimmter Substanzen, auch die olfaktorischen Eigenschaften chemischer Verbindungen an ganz bestimmte Molekülstrukturen gebunden sind mit der Folge, dass bereits geringe Abweichungen von einer solchen, charakteristischen Grundstruktur, zum völligen Verlust der erwünschten und wirtschaftlich genutzten Eigenart des chemischen Stoffes führen können.

Das als Alkoholkomponente bei der Gewinnung der neuen Riechstoffe verwendete 2-Methyl-2-sec.butyl-1,3-propandiol ist eine handelsübliche Verbindung, zu deren Herstellung verschiedene Synthesewege bekannt sind. Nach einem bewährten Verfahren geht man von 2-Ethylbutanol aus, das man zu 3-Methylpenten-2 dehydratisiert. Anschließend wird das Olefin unter Bildung von 2,3-Dimethylpentanal hydroformyliert und der Aldehyd durch Cannizzaro-Reaktion mit Formaldehyd in das Propandiol überführt. Die Reinigung des Reaktionsproduktes erfolgt durch fraktionierte Destillation.

Die als weitere Komponenten zur Herstellung der beanspruchten cyclischen Acetale genannten Formylverbindungen leiten sich von Tricyclo[5.2.1.0^{2.6}]decan ab. In diesen Cycloaliphaten ist jeweils ein Ringwasserstoffatom durch eine Formylgruppe ersetzt.

Die vorstehend beschriebenen Formylverbindungen werden überwiegend durch Hydroformylierung (Oxosynthese) der entsprechenden Olefine gewonnen. Man ist hierbei nicht an eine bestimmte Ausführungsform der Hydroformylierungsreaktion gebunden. Daher kann man die Formylverbindung aus Olefin und Wassergas sowohl nach der klassischen Variante der Umsetzung mit Cobalt-Katalysatoren bei hohem Druck erhalten, als auch nach modernen Verfahren bei geringem Druck in Gegenwart von (gegebenenfalls modifizierten) Rhodium-Katalysatoren gewinnen. 8(9)-Formyltricyclo[5.2.1.0^{2.6}]decen-3 ist das Ergebnis der Hydroformylierung von Dicyclopentadien, die anschließende partielle Hydrierung des 8(9)-Formyltricyclodecens-3 führt zum 8-Formyltricyclo[5.2.1.0^{2.6}]decan.

Die Herstellung der als Riechstoffe geeigneten Acetale erfolgt in bekannter Weise durch Umsetzung von Aldehyd und Diol in Gegenwart eines sauren Katalysators [vgl. z.B. Houben-Weyl; Methoden der organischen Chemie, Band E3 Seiten 615ff, (1983)]. Das molare Verhältnis der Reaktionspartner kann in weiten Bereichen variieren und beträgt üblicherweise (Aldehyd : Diol) 1:0,2 bis 1:5. Bevorzugt wendet man die Ausgangsstoffe im Molverhältnis 1:1,2 bis 1:0,8 und insbesondere 1:1 an. Als Katalysatoren werden ausschließlich saure Verbindungen eingesetzt, insbesondere technisch leicht verfügbare Säuren wie Schwefelsäure, p-Toluolsulfonsäure, Methansulfonsäure und Chlorwasserstoff. In Sonderfällen finden als Katalysatoren auch andere, sauer reagierende Verbindungen Anwendung, z.B. Ammoniumchlorid, Calciumchlorid, Zinkchlorid, Natriumhydrogensulfat und insbesondere Eisen(III)-chlorid (vgl. Houben-Weyl, I.c., Band VII, Teil 1, Seiten 417ff (1954)). Die Katalysatoren gelangen üblicherweise in Mengen von weniger als 1 Gew.-%, bezogen auf den Aldehyd, zum Einsatz. Die bevorzugt verwendeten Säuren wie Schwefelsäure und p-Toluolsulfonsäure werden in Mengen von 1 bis 50 mmol, insbesondere 1 bis 10 mmol je mol Aldehyd eingesetzt. Die Reaktionszeit, bestimmt durch Messung der abgeschiedenen Wassermenge (die theoretisch 1 mol Wasser je mol Acetal entspricht), beträgt im allgemeinen 1 bis 10 Stunden, in den meisten Fällen ist die Reaktion nach 1 bis 3 Stunden beendet. Die Reaktionstemperatur ist abhängig vom eingesetzten Aldehyd und des zur Entfernung des Wassers verwendeten Azeotropbildners (Schleppmittels). Als Schleppmittel haben sich insbesondere Kohlenwasserstoffe wie Cyclohexan, Toluol und Hexen-1 bewährt. In der Regel arbeitet man bei Normaldruck, die Anwendung geringen Unterdrucks (z.B. 500 hPa) ist jedoch nicht ausgeschlossen. Zur Aufarbeitung wird das Reaktionsgemisch gewöhnlich auf 20 bis 50°C abgekühlt und mit verdünnter wäßriger Alkalilösung z.B. Natronlauge, bis zur Einstellung eines pH-Wertes von etwa 6,5 bis 7,5, vorzugsweise 7,0, versetzt. Darauf trennt man wäßrige und organische Phase voneinander, wäscht die organische Phase mit Wasser und trennt erneut die Phasen. Anschließend reinigt man die organische Phase durch Destillation in konventionellen Apparaturen. Die Destillation kann bei Normaldruck erfolgen, wegen der hohen Siedepunkte der Acetale bevorzugt man jedoch verminderten Druck. Die Reinheit der so gewonnen Acetale liegt nach GC-Analyse im allgemeinen oberhalb 98%.

Die erfindungsgemäßen Acetale zeichnen sich durch einen angenehmen, leicht erdigen Geruch aus. Sie besitzen hohe Haftfestigkeit. Physiologische Unverträglichkeiten, d.h. Schädigungen am oder im Körper, sind nicht bekannt. Überdies sind sie beständig gegenüber Licht, Luft und das umgebende Medium. Sie können allein in unverdünnter Form oder auf Grund ihrer Löslichkeit oder Homogenisierbarkeit mit weiteren Komponenten verdünnt und gegebenenfalls zusammen mit üblichen Hilfsstoffen wie Fixateuren, angewandt werden. Mit anderen Riechstoffen in verschiedenen Mengenverhältnissen gemischt, ergeben sie neue Riechstoffkompositionen. Im allgemeinen beträgt ihr Anteil in derartigen Kompositionen 0,5 bis 5 Gew.-%, bezogen auf die gesamte Komposition. Die erfindungsgemäßen Riechstoffe und aus ihnen hergestellte Kompositionen können unmittelbar als Parfüm dienen oder auch zur Parfümierung von Kosmetika wie Cremes, Lotionen, Duftwässern, Aerosolen, Seifen, verwendet werden. Mit gleich gutem Erfolg können sie auch zur Geruchsverbesserung von technischen Produkten wie Wasch- und Reinigungsmitteln eingesetzt werden. Zur Parfümierung der verschiedenen Produkte werden die neuen Riechstoffe oder mit ihnen hergestellte Kompositionen in Konzentrationen von 0,05 bis 2 Gew.%, bezogen auf das Produkt-/ Riechstoffgemisch, angewandt.

In den nachfolgenden Beispielen wird ein Weg zur Herstellung der neuen Verbindungen näher beschrieben, selbstverständlich ist die Gewinnung der Acetale nicht auf diesen Syntheseweg beschränkt. In den Beispielen steht die Abkürzung MsBPD für die chemische Verbindung 2-Methyl-2-sec.butyl-1,3-propandiol. Die in Klammern beigefügten Ziffern I und II weisen auf die Formelbilder im Anschluss an die Beispiele hin.

### Beispiel 1

### Herstellung eines Gemisches von 2-(Tricyclo[5.2.1.0^{2.6}]-dec-3-en-8-yl)-5-methyl-5-(1-methylpropyl)-1,3-dioxan und 2-(Tricyclo[5.2.1.0^{2.6}]-dec-3-en-9-yl)-5-methyl-5-(1-methylpropyl)-1,3-dioxan (I)

In einem mit Rührer, Wasserabscheider und Thermometer ausgestatteten 41-Dreihalskolben werden 811,0 g (5,0 mol) eines bei der Hydroformylierung von Dicyclopentadien erhaltenen Gemisches von 8- und 9-Formyltricyclo[5.2.1.0^{2.6}]-decen-3 (TCD-Monenal), 731,0 g (5,0 mol) MsBPD, 1,9 g (10 mmol) p-Toluolsulfonsäure und 486,2 g Cyclohexan vorgelegt und unter Rühren erhitzt. Nach Ausschleusen der theoretischen Wassermenge (90 g) wird das Reaktionsgemisch auf 30°C abgekühlt und mit Wasser gewaschen. Nach Trennung der Phasen und nach destillativer Aufarbeitung des Reaktionsgemisches über eine Kolonne mit 4,5 theoretischen Böden erhält man 1 121 g des Gemischs der Acetale, das bei 5 mbar (500 Pa) im Bereich von 170 bis 175°C siedet; seine Reinheit beträgt 99,6%, die Ausbeute 76,9% d.Th.

### Beispiel 2

### Herstellung von 2-(Tricyclo[5.2.1.0^{2.6}]-dec-8-yl)-5-methyl-5-(1-methylpropyl)-1,3-dioxan (II)

Analog Beispiel 1 werden 821,0 g (5,0 mol) 8-Formyl-tricyclo[5.2.1.0^{2.6}]-decan, und 731,0 g (5,0 mol) MsBPD in Gegenwart von 1,9 g (10 mmol) p-Toluolsulfonsäure und 490 g Cyclohexan umgesetzt. Nach destillativer Aufarbeitung des Reaktionsgemisches erhält man 1 272,0 g des Acetals, das bei 5 mbar (500 Pa) im Bereich von 166 bis 171°C siedet; seine Reinheit beträgt 99,5,%, die Ausbeute 86,6% d.Th.

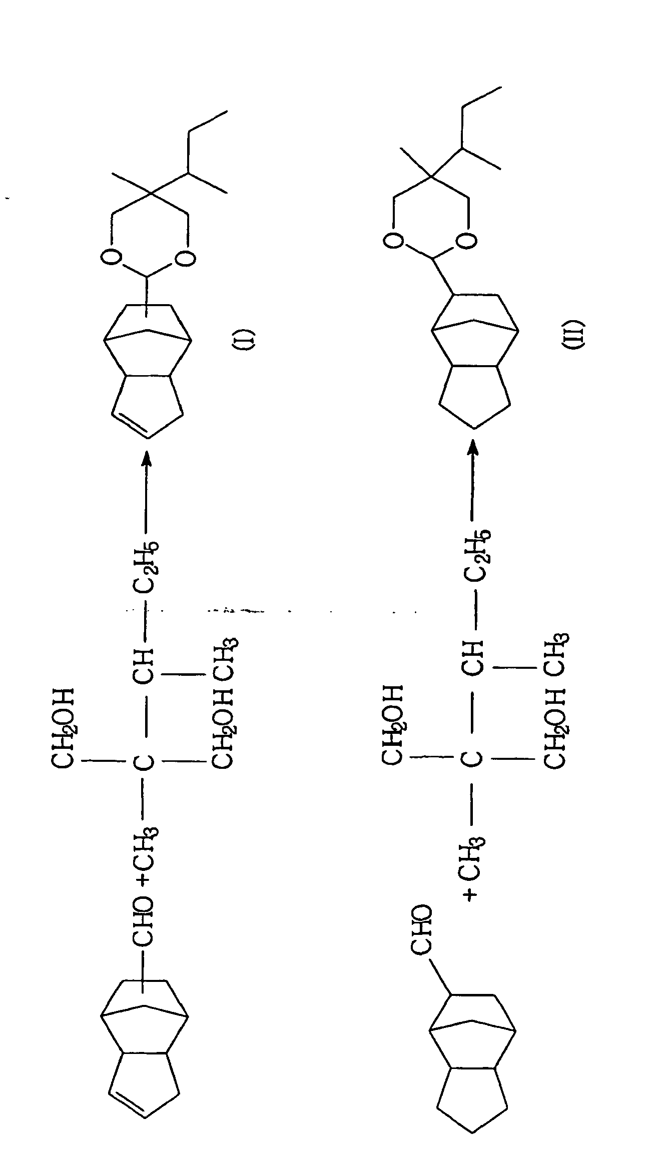

## Patentansprüche

1. Riechstoffe auf Basis der chemischen Verbindung 2-(Tricyclo[5.2.1.0^{2.6}]-dec-8-yl)-5-methyl-5-(1-methylpropyl)-1,3-dioxan oder auf Basis eines Gemisches bestehend aus 2-(Tricyclo[5.2.1.0^{2.6}]-dec-3-en-8-yl)-5-methyl-5-(1-methylpropyl)-1,3-dioxan und 2-(Tricyclo[5.2.1.0^{2.6}]-dec-3-en-9-yl)-5-methyl-5-(1-methylpropyl)-1,3-dioxan.

2. Verfahren zur Herstellung von Riechstoffen nach Anspruch 1, **dadurch gekennzeichnet**, d ass m an 8-Formyl-tricyclo[5.2.1.0^{2.6}]-decan o der ein Gemisch von 8- und 9-Formyl-tricyclo[5.2.1.0^{2.6}]-decen-3, mit 2-Methyl-2-sec.butyl-1,3-propandiol im Molverhältnis 1:0,2 bis 1:5 in Gegenwart eines sauren Katalysators und eines Schleppmittels zur Entfernung des bei der Reaktion entstehenden Wassers bei erhöhter Temperatur umsetzt, das Reaktionsgemisch auf 20 bis 50°C abkühlt, darauf verdünnte wässrige Alkalilösung bis zur Einstellung eines pH-Wertes von etwa 6,5 bis 7,5 zufügt, anschließend wässrige und organische Phase voneinander trennt, die organische Phase mit Wasser wäscht, erneut von der wässrigen Phase trennt und durch Destillation reinigt.

3. Riechstoffkompositionen enthaltend Riechstoffe nach Anspruch 1.

## Claims

1. A fragrance based on the chemical compound 2-(tricyclo[5.2.1.0^{2.6}]-dec-8-yl)-5-methyl-5-(1-methylpropyl)-1,3-dioxane or based on a mixture consisting of 2-(tricyclo[5.2.1.0^{2.6}]-dec-3-en-8-yl)-5-methyl-5-(1-methylpropyl)-1,3-dioxane and 2-(tricyclo[5.2.1.0^{2.6}]-dec-3-en-9-yl)-5-methyl-5-(1-methylpropyl)-1,3-dioxane.

2. A method of preparing fragrances as claimed in claim 1, wherein 8-formyltricyclo[5.2.1.0^{2.6}]decane or a mixture of 8- and 9-formyltricyclo[5.2.1.0^{2.6}]decene-3 is reacted with 2-methyl-2-sec-butyl-1,3-propanediol in the molar ratio 1:0.2 to 1:5 in the presence of an acidic catalyst and an entrainer for removing the water which forms during the reaction at elevated temperature, the reaction mixture is cooled to 20 to 50°C, dilute aqueous alkali solution is added thereto in order to establish a pH of from about 6.5 to 7.5, then aqueous and organic phases are separated from one another, the organic phase is washed with water, separated again from the aqueous phase and purified by distillation.

3. A fragrant composition comprising a fragrance as claimed in claim 1.

## Revendications

1. Principes odorants à base du composé chimique 2-(tricyclo[5.2.1.0^{2.6}] -déc-8-yl)-5-méthyl-5-(1-méthylpropyl)-1,3-dioxane ou à base d'un mélange constitué de 2- (tricyclo[5.2.1.0^{2.6}]-déc-3-én-8-yl)-5-méthyl-5-(1-méthylpropyl)-1,3-dioxane et de 2-(tricyclo[5.2.1.0^{2.6}] -déc-3-én-9-yl)-5-méthyl-5-(1-méthylpropyl)-1,3-dioxane.

2. Procédé de préparation de principes odorants selon la revendication 1, **caractérisé en ce que** l'on fait réagir du 8-formyl-tricyclo[5.2.1.0^{2.6}]-décane ou un mélange de 8- et 9- formyltricyclo[5.2.1.0^{2.6}]-décène-3, avec du 2-méthyl-2-sec-butyl-1,3-propanediol en un rapport molaire de 1 : 0,2 à 1 : 5, à une température élevée, en présence d'un catalyseur acide et d'un agent d'entrainement en vue d'éliminer l'eau produite lors de la réaction, on refroidit le mélange réactionnel jusqu'à 20-50°C, puis on ajoute une solution alcaline aqueuse diluée, on sépare ensuite les phases aqueuse et organique l'une de l'autre, on lave la phase organique à l'eau, on la sépare à nouveau de la phase aqueuse et on purifie par distillation.

3. Compositions de principes odorants contenant des principes odorants selon la revendication 1.
